Europäisches Patentamt

European Patent Office

Office européen des brevets

(19)

(11) Veröffentlichungsnummer: **0 068 149**
A2

## (12) EUROPÄISCHE PATENTANMELDUNG

(21) Anmeldenummer: 82104606.7

(22) Anmeldetag: 26.05.82

(51) Int. Cl.³: **A 61 L 15/04,** A 61 L 15/03,
A 61 L 17/00, A 61 K 37/54,
A 61 K 37/02

(30) Priorität: 25.06.81 DE 3124933
25.06.81 DE 3124962
12.08.81 DE 3131827
18.12.81 EP 81110615

(43) Veröffentlichungstag der Anmeldung: 05.01.83
Patentblatt 83/1

(84) Benannte Vertragsstaaten: **AT BE CH DE FR GB IT LI LU NL SE**

(71) Anmelder: **Serapharm - Michael Stroetmann,
Kaiser-Wilhelm-Ring 36, D-4400 Münster (DE)**

(72) Erfinder: **Stroetmann, Michael, Kaiser-Wilhelm-Ring 36,
D-4400 Münster (DE)**

(74) Vertreter: **Brehm, Hans-Peter, Dr. Dipl.-Chem. et al,
Patentanwälte Tischer, Kern & Brehm
Albert-Rosshaupter-Strasse 65, D-8000 München 70 (DE)**

(54) Fibrinogen-haltiges Trockenpräparat, dessen Herstellung und Verwendung.

㉟ Ein Trockenpräparat mit einer durch Gefriertrocknung erzielten Schaum- bzw. Vliesstruktur besteht neben Thrombin in zumindest katalytisch wirksamen Mengen im wesentlichen aus etwa 10 bis 95 Gew.-% Fibrin und etwa 5 bis 90 Gew.-% Fibrinogen. Zur Herstellung wird in einer Fibrinogen und Thrombin enthaltenden wäßrigen Lösung in situ Fibrin erzeugt, und das entstehende Reaktionsgemisch tiefgefroren und lyophilisiert. Als weitere Bestandteile des Trockenpräparates kommen Wirkstoffe wie etwa Antibiotika, natürliches Knochenmaterial und/oder synthetischer, knochenbildender Ersatzstoff, Glykoproteine, gerinnungsfördernde Substanzen und dergleichen, und/oder Fibrinolyse-Inhibitoren in Betracht. Das Trockenpräparat ist vor allem als Wundversorgungsmaterial, Füllmaterial für Knochenhohlräume und/oder als Trägermaterial für weitere Wirkstoffe vorgesehen.

0068149

# Fibrinogen-haltiges Trockenpräparat, dessen Herstellung und Verwendung

Die Erfindung betrifft ein Fibrinogen-haltiges Trockenpräparat mit einer durch Gefriertrocknung erzielten Schaum- bzw. Vliesstruktur. Ein solches, vollständig resorbierbares Trockenpräparat kann insbesondere als Wundversorgungsmaterial, als Füllmaterial für pathologische Knochenhohlräume und/oder als Trägermaterial für weitere, einen Heilprozeß fördernde Wirkstoffe verwendet werden. Weiterhin betrifft die Erfindung ein Verfahren zur Herstellung dieses Trockenpräparates und dessen Anwendung.

Ein Trockenpräparat der genannten Art ist aus der DE-OS 30 37 513 bekannt. Hierbei handelt es sich um eine collagene Wundauflage, die durch Gefriertrocknung einer sowohl Collagen wie Fibrinogen enthaltenden Lösung erhalten worden ist. Das gebildete Trockenmaterial kann zusätzlich einen Arzneimittelwirkstoff, etwa ein Antibiotikum, enthalten. Ein erheblicher Collagengehalt in einer Wundauflage - insbesondere wenn diese resorbiert werden soll - ist nicht unbedenklich, wie nachstehend ausgeführt wird.

Ferner ist ein lyophilisierter Gewebekleber bekannt (vgl. DE-OS 30 02 934), der im wesentlichen - neben Fibrinolyse-Inhibitor und Faktor XIII - aus Fibrinogen und Albumin besteht und zusätzlich Glycin, Glucose und Heparin enthalten kann. Dieser Gewebekleber soll jedoch nicht in Form des Trockenpräparates auf der Wunde aufgebracht werden, sondern durch Zugabe von Wasser rekonstituiert werden, um eine konzentrierte Fibrinogen-Lösung zu erhalten, die daraufhin mit Thrombin und $CaCl_2$ versetzt wird.

Weiterhin ist mit der DE-OS 28 52 319 eine absorbierbare haemostatische Masse zur Verwendung bei der Bekämpfung von Knochenhaemorrhagien bekanntgeworden, die in einer wasserlöslichen, bioverträglichen Grundlage ein haemostatisches Pulver, nämlich ein Gemisch aus Fibrin- und Collagenpulver aufweist.

Schließlich sind aus der US-PS 3 523 807 nach Implantation vollständig resorbierbare Formkörper bekannt, die im wesentlichen aus Fibrin bestehen. Zur Herstellung wird Fibrin aus Plasma durch Calciumchlorid-Zugabe abgetrennt, der Niederschlag getrocknet und pulverisiert, das erhaltene Pulver gegebenenfalls mit anderem pulverförmigen Protein vermischt, das Pulver mit Wasser angeteigt und in einer Form zu dem gewünschten Formkörper gepreßt, der danach in einem Formaldehyd enthaltenden Bad gehärtet wird.

Die Gefriertrocknung (Lyophilisation) wässriger Protein-Lösungen ist in der Fachwelt bekannt. Man erhält eine lockere Schaum-, Filz- oder Vliesstruktur mit zahlreichen Hohlräumen, was zur Folge hat, daß ein derartiges Trockenpräparat eine große Saugfähigkeit für Körperflüssigkeiten aufweist. Die Herstellung eines Collagenproduktes mit filz- bzw. vliesartiger Faserstruktur durch Gefriertrocknung einer wässrigen Collagen-Lösung ist beispielsweise in der DE-OS 26 25 289 beschrieben. Derartige Collagen-Trockenpräparate zeichnen sich durch eine hohe mechanische Festigkeit aus, so daß man solche Collagenvliese schneiden und biegen kann.

Würde man dagegen unter vergleichbaren Bedingungen eine wässrige Fibrinogen-Lösung durch Gefriertrocknung zu einem Trockenpräparat verarbeiten, so würde man eine Proteinplatte mit schaumartiger Struktur erhalten, die leicht bricht und zerkrümelt. Wegen ihrer unzureichenden mechanischen Festigkeit wäre eine solche Platte als Wundversorgungsmaterial und/oder Trägermaterial für Arzneimittel-Wirkstoffe nicht geeignet.

Trotz ihrer haemostatischen Wirkung und hohen Stabilität (mechanische Festigkeit, Lagerfähigkeit) haben gefriergetrocknete und andere Collagenprodukte bei der Anwendung als Wundversorgungsmaterial und/oder als resorbierbares Implantat in der Praxis nicht völlig befriedigt. Nachteilig ist beispielsweise die lange Verweildauer von bis zu 6 Wochen und mehr im Wundgebiet und unerwünschte Auswirkungen von Collagen auf bestimmte Heilungsprozesse, beispielsweise bei Knochenfrakturen. Bekannte Collagenpräparate sind häufig im Verlauf ihrer Abtrennung aus natürlichem Material und Zubereitung denaturiert worden, so daß ein unlösliches Präparat mit schlechtem Resorptionsverhalten resultiert. Im Gegensatz dazu enthalten leicht lösliche Collagenpräparate stets einen sauren pH-Wert erzeugende Salze. Nach Einbringen in eine Wunde erzeugen solche Collagenpräparate dort wiederum eine saure Umgebung, deren Neutralisierung den Organismus unnötig belastet und den Heilungsprozeß beeinträchtigt. Gerade die Wundheilung und die durch Thrombin induzierte Fibrinbildung aus vorhandenem löslichen Fibrinogen benötigen für den optimalen Verlauf eine Umgebung mit physiologischem pH-Wert von etwa 7,36. Schließlich ist Collagen im Wundgebiet in den körpereigenen Strukturen, Geweben und Flüssigkeiten schon in physiologisch verwertbarer Form vorhanden, so daß ein erhebliches zusätzliches Collagenangebot weder notwendig noch wünschenswert ist.

Natürliches Wundverschlußmaterial stellt ein aus Fibrinogen gebildetes Fibrin in gelartigem Zustand dar, das immer noch einen großen Anteil an nativem Fibrinogen enthält. Die in vitro Bereitstellung eines Trockenpräparates, das dem natürlichen Wundverschlußmaterial mehr angeglichen ist, als herkömmliche Collagen-Trockenpräparate, und das dennoch die vorteilhaften Eigenschaften solcher Collagenpräparate aufweist, wie etwa hohe mechanische Festigkeit, gute Saugfähigkeit und großes Flüssigkeits-Festhaltevermögen sowie unbegrenzte Lagerfähigkeit bei Raumtemperatur, würde zu einer erheblichen Verbesserung der medizinischen Versorgung führen.

Davon ausgehend besteht die Aufgabe der vorliegenden Erfindung darin, ein Fibrinogen-haltiges Trochenpräparat der oben angegebenen Art bereitzustellen, das völlig oder weitgehend Collagen-frei ist und dennoch die vorteilhaften Eigenschaften üblicher Collagenpräparate aufweist, das hinsichtlich seiner Zusammensetzung, seiner physiologischen Eigenschaften und seines Resorptionsverhalten besser an natürliches Wundverschlußmaterial angeglichen ist und das unmittelbar, d.h. ohne weitere Manipulationen, wie etwa die Zugabe von zusätzlichen und/oder aktivierenden Komponenten auf der Wunde applizierbar ist und dort eine beschleunigte Haemostase bewirkt.

Die erfindungsgemäße Lösung dieser Aufgabe ist ein durch Gefriertrocknung erhaltenes Vlies, das neben Thrombin in zumindest katalytisch wirksamen Mengen im wesentlichen aus etwa 10 bis 95 Gew.-% Fibrin und etwa 5 bis 90 Gew.-% Fibrinogen besteht.

Das erfindungsgemäße Verfahren zur Herstellung eines solchen Trockenpräparates sieht vor, in einer Fibrinogen und Thrombin enthaltenden wässrigen Lösung in situ Fibrin zu erzeugen, und das entstehende Reaktionsgemisch tiefzufrieren und zu lyophilisieren.

Ein weiterer Gesichtspunkt der Erfindung betrifft die Verwendung eines solchen Trockenpräparates zur akzelerierten Haemostase und optimierten biochemischen Regelung des Wundverschlußes sowie zur Behandlung von Knochenmarksentzündungen. Weiterhin kann das Trockenpräparat für die verschiedensten Anwendungen als Trägermaterial bzw. als Wirkstoffdepot für irgendwelche, einen Heilungsprozeß fördernde Wirkstoffe dienen. Die Wirkstoff-Freisetzung aus dem Trockenpräparat kann hinsichtlich Menge und Dauer durch seinen Fibringehalt, durch zusätzliche Bestandteile, sowie durch einen bestimmten Vernetzungs- und/oder Polymerisationsgrad des Fibrins gesteuert werden.

Vorteilhafte Ausgestaltungen und Weiterbildungen der Erfindung ergeben sich aus den Unteransprüchen.

Die Erfindung beruht auf der Beobachtung, daß durch Gefriertrocknung eines Reaktionsgemisches, das neben Fibrinogen und zumindest katalytischen Mengen Thrombin in situ gebildetes Fibrin enthält, ein Trockenpräparat erhalten wird, das eine überraschend hohe Festigkeit, vergleichbar mit der Festigkeit gefriergetrockneter Collagenprodukte, aufweist. Ein solches Trockenpräparat weist auch ohne den Zusatz weiterer, die Blutgerinnung fördernder Substanzen und Faktoren eine besonders hohe haemostatische Aktivität auf, insbesondere wegen des zusätzlichen Angebotes an wirksamem Thrombin und Fibrinogen. Das mit dem Trockenpräparat angebotene, in situ gebildete Fibrin ist vorzugsweise lediglich in seiner Längsrichtung vernetztes, also im wesentlichen nur über seine $\gamma$-Ketten verknüpftes, insbesondere dimerisiertes Fibrin hoher biologischer Aktivität, das als Ansatzpunkt für eine weitere und verstärkte Fibrinbildung im Wundbereich dient. Mit dem erfindungsgemäßen Trockenpräparat kann ein Komponentengemisch angeboten werden, das große Ähnlichkeit mit dem natürlichen Wundverschlußmaterial aufweist, das deshalb vom Organismus ohne weiteres angenommen und vollständig resorbiert wird. Ein erfindungsgemäß als Wundversorgungsmaterial konfektioniertes Trockenpräparat, etwa in der Form eines 6 bis 20 mm starken Vlieses, vermag auch starke Blutungen in kurzer Zeit, etwa in 2 Min. zum Stillstand bringen.

Zur Herstellung des erfindungsgemäßen Trockenpräparates geht man von eine wässrigen oder überwiegend wässrigen Lösung aus. Bei dieser Ausgangslösung kann es sich um Wasser, Human-Serum oder um eine wässrige Salzlösung handeln. Die wässrige Salzlösung kann eine physiologische Kochsalzlösung sein, oder
eine physiologische Kochsalzlösung, die zusätzlich mit $CaCl_2$
und Phosphatsalzen angereichert worden ist. Der pH-Wert und
der Salzgehalt dieser Ausgangslösung soll den physiologischen
Bedingungen weitgehend entsprechen. In manchen Fällen kann
es zweckmäßig sein, eine überwiegend wässrige Lösung zu verwenden, die neben Wasser wasserlösliche, organische Lösungsmittel enthält, beispielsweise, um die Löslichkeit für bestimmte Arzneimittel-Wirkstoffe zu erhöhen. Zu geeigneten
organischen Lösungsmitteln gehören einwertige Alkohole wie
Äthanol oder Isopropanol, mehrwertige Alkohole wie Glycerin
oder Polyglykole, cyclische Äther wie Dioxan und dg.. Der
maximale Anteil an organischem Lösungsmitteln wird dahingehend ausgewählt, daß jegliche Denaturierung und/oder Aktivitätsabnahme der eingesetzten Enzyme unterbleibt und die
Trocknung der gefrorenen Masse nicht beeinträchtigt wird.
Zumeist soll der Anteil an organischem Lösungsmittel 20 %
des Volumens des Gesamtansatzes nicht übersteigen.

Die Ausgangs-Lösung wird mit Human-Fibrinogen versetzt. Geeignete Präparate sind handelsüblich zugänglich und können
beispielsweise von der Firma Behring-Werke, Marburg, bezogen
werden. Ein gut geeignetes Fibrinogen-Präparat kann ferner
entsprechend nachstehendem Verfahren erhalten werden.

Human-Plasma wird auf 4°C abgekühlt und unter Rühren mit
ß-Alanin (2 molare Lösung in Äthanol) versetzt, bis unter
weiterer Äthanol-Zugabe das Rohfibrinogen ausfällt. Dieses
Rohfibrinogen wird abzentrifugiert, in 0,01 M Tris-Puffer
(pH-Wert 7,4) gelöst und durch Zusatz von 2 M Glycin erneut
gefällt. Das abgetrennte Sediment wird in 0,9%-iger wässriger
NaCl-Lösung gelöst, gegen das gleiche Lösungsmittel dialysiert, entsalzt und anschließend lyophilisiert. Das erhaltene
mikrokristalline Fibrinogen weist ein Molekulargewicht von
340 000 $\pm$ 5 % auf, ist in der $\alpha$-Kette leicht angedaut,
löst sich nach Einbringen in Körperflüssigkeit schnell, und
beginnt unmittelbar darauf zu polymerisieren. Der in Lösung gerinnbare Anteil des Fibrinogens macht wenigstens 85 % aus.

Die oben genannte, vorzugsweise bei Raumtemperatur gehaltene Ausgangs-Lösung wird pro 1 ml Lösung mit etwa 10 bis 90 mg Fibrinogen versetzt. Vorzugsweise ist eine relativ hohe Fibrinogen-Konzentration von etwa 50 bis 80 mg/ml vorgesehen. Sofern eine Verschäumung der Lösung unterbleibt, weist das nach der Gefriertrocknung erhaltene Präparat praktisch das Volumen der Ausgangs-Lösung auf. Eine höhere Fibrinogen-Konzentration in der Ausgangs-Lösung führt daher zu einem dichteren Endprodukt mit höherer mechanischer Festigkeit.

Das zugesetzte Fibrinogen wird in der Ausgangslösung gelöst, wozu bei Raumtemperatur gerührt werden kann.

Nach einem alternativen Verfahren ist es nicht erforderlich, zuerst das Fibrinogen in reiner, fester Form zu isolieren, und daraufhin einer wässrigen Lösung zuzusetzen. Beispielsweise kann das bei der oben beschriebenen Fibrinogen-Gewinnung beschriebene, nach der Glycin-Fällung erhaltene Sediment in 0,9 %-iger, wässriger NaCl-Lösung gelöst und auf den gewünschten Konzentrationsgrad eingestellt werden, und unmittelbar zu dieser Lösung die weiteren Zusätze hinzugefügt werden, wie das nachstehend ausgeführt ist. Auch andere Fibrinogen-Lösungen sind geeignet, zu deren Herstellung das Rohfibrinogen als Kryopräzipitat vom restlichen Serum mit seinen Proteinen und Faktoren abgetrennt worden ist, beispielsweise entsprechend dem Verfahren nach der DE-OS 30 02 934. Wichtig ist eine weitgehende Abtrennung der die spontane Fibrinbildung auslösenden Enzyme und/oder Faktoren, so daß in der Ausgangslösung ein erheblicher Gehalt an stabilisierend wirkenden Salzen wie Citrat, Phosphat, Oxalat oder dgl. vermieden wird. Gerade das als Wundversorgungsmaterial vorgesehene Trockenpräparat soll ein Komponentengemisch liefern, das dem natürlichen Wundverschlußmaterial weitgehend angeglichen ist. Insbesondere für diese Anwendung sollen un-physiologische Salzkonzentrationen in der Fibrinogen-Ausgangs-Lösung vermieden werdem. Andererseits kann bei der Bereitstellung dieser Ausgangslösung bereits auf eine Anreicherung an Gerinnungsfaktoren, etwa Faktor XIII hingearbeitet werden. Der Fibrinogengehalt einer solchen Fibrinogen-Ausgangslösung soll ebenfalls 10 bis 90 mg, vorzugsweise 50 bis 80 mg Fibrinogen pro 1 ml Lösung betragen.

Die nach dem einen oder anderen Verfahren erhaltene Fibrinogen-Lösung kann sterilisiert werden, insbesondere gezielt zur Inaktivierung von Viren wie etwa Hepatitis-Viren, behandelt werden. Beispielsweise kann hierzu eine Behandlung mit ß-Propiolacton, gefolgt von einer UV-Bestrahlung, vorgesehen werden. Alternativ kann mit energiereicher $\beta$- oder Röntgenstrahlung sterilisiert werden.

Anschließend können der Fibrinogen-Lösung die wahlweise vorgesehenen Zusätze und Wirkstoffe hinzugefügt werden.

Die Biegsamkeit, mechanische Festigkeit und Stabilität des Trockenpräparates kann durch die Beimischung verschiedener Glykoproteine gesteuert werden. Hierzu kann der Fibrinogen-Lösung ein oder mehrere Glykoproteine wie Albumin, Lipoprotein, Fibronektin und/oder Globulin ($\alpha$-, $\beta$-, $\gamma$-Globuline) zugesetzt werden. Für diese Glykoproteine kann ein Anteil von etwa 3 bis 40, vorzugsweise von etwa 5 bis 25 Gew.-% des fertigen Trockenpräparates vorgesehen werden. Derartige Glykoproteine dienen auch als Trockenhalte- und Stabilisierungsmittel und beugen einem Aktivitätsverlust der Gerinnungsenzyme während langer Lagerdauer vor.

Sofern das Trockenpräparat gezielt als Trägermaterial oder Depotstoff für bestimmte Wirkstoffe dienen soll, können diese, sofern sie wasserlöslich sind, und durch die Gefriertrocknung nicht geschädigt werden, ebenfalls der Fibrinogen-Lösung zugesetzt werden. Der Ausdruck "Wirkstoff" wird in einem sehr breiten Sinn verstanden und umfaßt alle Zusammensetzungen, die auf parenteralem Wege zur Heilung, Linderung, Behandlung und/oder Verhinderung von Gesundheitsstörungen bei Mensch und Tier wirksam sind oder die Funktion des Organismus zu beeinflussen vermögen. Vor allem kommen hier antibakterielle Wirkstoffe, insbesondere Antibiotika, in Betracht. Zu geeigneten Antibiotika gehört etwa die Gruppe der Aminoglykosid-Antibiotika, wie etwa Gentamycin, die Lacton-Antibiotika wie etwa Novobiocyn, die Gruppe der Penicilline wie etwa Baycillin oder Amoxycillin, das Chloramphenicol und dessen Derivate, wie etwa Tiamphenicol und sonstige Antibiotika, wie etwa die Gruppe der Streptomycine, die Gruppe der Tetracycline und dgl.. Zu weiteren geeigneten antibakteriellen Wirkstoffen gehören beispielsweise die Sulfonamide. Wegen ihres breiten Wirkungsspektrums sind die Aminoglykosid-Antibiotika, hier insbesondere Gentamycin, besonders bevorzugt. Weiterhin kann eine Kombination verschiedener Antibiotika, beispielsweise Gentamycin, zusammen mit Tetracyclin, vorgesehen werden. Zu weiteren, geeigneten Wirkstoffen anderer Indikation gehören die Antiseptika wie etwa Salicylsäure oder Undecensäure, ferner die Entzündungshmmer wie etwa Pyrazolone und ferner die Cytostatika wie etwa Prednison.

Die Menge dieser Wirkstoffe kann in weiten Bereichen schwanken und hängt hauptsächlich von der Wirkstoffaktivität ab. Bezogen auf das Gesamtgewicht des Trockenproduktes kann der Wirkstoffgehalt etwa 0,1 bis 10 Gew.-%, vorzugsweise etwa 2 bis 6 Gew.-% betragen. Beispielsweise hat sich ein Gentamycin-Zusatz von etwa 500 bis 10 000 Einheiten pro 1 ml Fibrinogen-Lösung gut bewährt.

Sofern das Trockenpräparat gezielt in Knochenhöhlen eingebracht werden soll, um dort als Wirkstoffdepot mit zeitlich und mengenmäßig gezielter Wirkstoff-Freisetzung zu dienen und zugleich die Knochennachbildung zu fördern, können gezielt das Knochenwachstum fördernde Zusätze vorgesehen werden. Hierzu gehören beispielsweise natürliches feinteiliges Knochenmaterial wie denaturiertes Knochenmehl oder lyophilisierte Knochenpartikel, die anschließend pulverisiert worden sind, und/oder synthetischer, knochenbildender Ersatzstoff, wie etwa anorganische Salze des Kaliums, Magnesiums und und Calciums, insbesondere Calciumphosphat und hier besonders bevorzugt Tricalciumphosphat. Für diesen besonderen Anwendungszweck kann der Anteil an das Knochenwachstum fördernden Zusätzen relativ hoch gewählt werden und vorzugsweise etwa 50 Gew.-% des fertigen Trockenpräparates ausmachen.

In Verbindung mit den genannten Wirkstoffen kann ein Zusatz vorgesehen werden, welcher die Wirkstoff-Freisetzung zeitlich und mengenmäßig steuert. Zu wirksamen Zusätzen dieser Art gehören Collagen und vernetztes Fibrin. Die Wirksamkeit dieser Zusätze hängt von der Affinität zu dem ausgewählten Wirkstoff ab. Für zahlreiche Wirkstoffe, insbesonder die wichtige Gruppe der Antibiotika und hier insbesondere Gentamycin, hat sich ein Anteil von die Wirkstoff-Freisetzung steuernden Zusätzen von etwa 2 bis 12 Gew.-%, vorzugsweise von etwa 4 bis 10 Gew.-%, bezogen auf das Gesamtgewicht des Trockenpräparates, als ausreichend erwiesen. Besonders bevorzugt wird zu diesem Zweck ein Collagengehalt von etwa 0,5 bis 1 Gew.-%. Zu diesem Zweck kann der Fibrinogen-Lösung in geeigneter Menge wasserlösliches Collagen zugesetzt oder das Fibrinogen in einer entsprechenden wässrigen Collagen-Lösung gelöst werden. Derartige Collagenmengen sind in dem Gesamtansatz ohne weiteres löslich, so daß die oben genannten Schwierigkeiten (saurer pH-Wert und/oder un-physiologische Salzkonzentrationen) nicht auftreten. Weiterhin kann die Wirkstoff-Freisetzung durch den Fibringehalt und/oder das Ausmaß der Fibrinvernetzung beeinflußt werden. Zumeist verzögern ein höherer Fibringehalt sowie eine stärkere Fibrinvernetzung die Wirkstoff-Freisetzung. Eine geeignete Fibrinvernetzung kann beispielsweise durch Zusatz von Glutaraldehyd bewirkt werden.

Sofern das Trockenpräparat gezielt als Wundversorgungsmaterial eingesetzt werden soll, kann ein gefriergetrocknetes Vlies mittlerer haemostatischer Wirksamkeit erzeugt werden, dem nachträglich ein hochwirksames, angereichertes, pulverförmiges Plasmaderivat zur akzelerierten Haemostase und optimierten Regelung des Wundverschlusses zugesetzt wird,

0068149

wie das nachstehend noch im einzelnen ausgeführt wird. Alternativ können bereits der Fibrinogen-Lösung die wesentlichen Komponenten dieses angereicherten Plasmaderivates zugesetzt werden. Zu den Hauptbestandteilen dieses Plasmaderivates gehören Fibrinogen, Thrombin, Komponenten des Prothrombinkomplexes und Protease-Inhibitoren; ferner können Beimischungen von Blutplättchenextrakten, Antibiotika und dgl. vorgesehen werden. Mehr im einzelnen kann zu diesem Zweck ein Zusatz von Phospholipiden, Prostaglandinen, Gerinnungsfaktoren, Antishisstaminika, Vasopressinen, Wachstumsfaktoren, Vitaminen, und dgl. vorgesehen werden. Die Anwesenheit von Prostaglandinen fördert die Aktivierung des Kapillarbettes im Wundgebiet, sowie die Aktivierung der im Blutstrom befindlichen Plättchen. Die Blutgerinnungsfaktoren, beispielsweise Faktor XIII, Blutplättchen-Extrakte und andere, zur Blutgerinnung notwendigen Faktoren wie etwa Leukotriene, Plättchen-aktivierender Faktor, unterstützen und verstärken die Wirkung der in der Körperflüssigkeit vorhandenen Faktoren im Sinne einer akzelerierten Haemostase und einer Optimierung des Wundverschlusses. Als Phospholipid dient vorzugsweise ein aus humanem Vollblut gewonnener Thrombozytenextrakt. Weitere geeignete Phospholipide sind etwa Extrakte aus Gehirnsubstanz. Die Gerinnungsfaktoren VIII und IX dienen zur haemophilen Wundversorgung. Ein Zusatz von Adrenalin und/oder Ergotamin wirkt gefäßaktiv, was im Ergebnis zu einer schnelleren Blutgerinnung führt. Im Hinblick auf ihre hohe spezifische Wirksamkeit macht die Summe der Anteile an Prostaglandinen, Phospholipiden, Gerinnungsfaktoren und den weiteren genannten Wirkstoffen zumeist nicht mehr als 1,2 Gew.-%, vorzugsweise nicht mehr als 0,8 Gew.-% des fertigen Trockenpräparates aus. Die genannten Substanzen in den genannten Mengen werden ebenfalls der Fibrinogen-Lösung, vor oder gemeinsam mit der Thrombinzugabe, hinzugefügt.

Ferner kann ein Fibrinolyse-Inhibitor zugesetzt werden. Vorzugsweise ist ein relativ hoher Anteil an Fibrinolyse-Inhibitor vorgesehen, nämlich wenigstens 5000 Einheiten (sog. KIE, nämlich Kalikrein-Inaktivator-Einheiten), vorzugsweise jedoch 10 000 und mehr Einheiten Fibrinolyse-Inhibitor pro 1 ml Lösung. Als Fibrinolyse-Inhibitor kommen beispielsweise Plasminogen-Aktivator-Inhibitor oder ein oder mehrere Antiplasmine, wie etwa $\alpha_1$-Antiplasmin, $\alpha_2$-Makroglobulin oder Aprotinin, sowie $\epsilon$-Aminocapronsäure und/oder Trypsin-Inhibitor in Betracht. Gut bewährt hat sich beispielsweise der von der Bayer AG, Leverkusen, unter der Handelsbezeichnung "Trasylol" vertriebene natürliche Fibrinolyse-Inhibitor.

Nachdem einer oder mehrere der genannten, oder andere, auf einen bestimmten Anwendungszweck hin ausgewählte Wirkstoffe der Fibrinogen-Lösung zugesetzt wurden und darin vollständig oder weitgehend gelöst worden sind, erfolgt die Zugabe von Thrombin. Bekanntlich vermag Thrombin von einem Fibrinogen-Molekül die Fibrinopeptide A und B abzuspalten, wodurch ein Fibrin-Monomer entsteht, das sich dann spontan mit anderen Fibrin-Monomeren umsetzt, so daß schließlich ein Polymerisat aus Fibrinmolekülen entsteht. Das zugesetzte Thrombin soll unter bekannten, standardisierten Bedingungen wenigstens eine biologische Aktivität von 10 000 Einheiten (NIH-Einheiten gemäß dem Standard des National Institut of Health der U.S.A.) pro 1 mg Thrombin aufweisen. Geeignete Präparate sind handelsüblich zugänglich. Beispielsweise kann geeignetes Thrombin in mikrokristalliner Form mit einer biologischen Aktivität von wenigstens 3 000 Einheiten/mg Präparat (das neben Thrombin bekannte Stabilisierungsmittel und Trägermaterialien enthält) unter der Handelsbezeichnung "Topostasin" von Hoffmach LaRoche, Grenzach, Baden, bezogen werden.

Ein Teil der gewünschten Thrombinaktivität kann auch in Form von Thrombin-bildenden Vorstufen, wie etwa Prothrombin, zugesetzt werden. Auch Prothrombin-Konzentrate sind handelsüblich zugänglich, beispielsweise als PPSB-Präparat der Firma Immuno AG, Wien. Ferner kann ein kombiniertes, Thrombin und Prothrombin enthaltendes Präparat durch Säulenchromatographie aus käuflichem Prothrombinkomplex abgetrennt oder aus Human-Plasma durch Bariumsulfat extrahiert und aus dem kristallinen Niederschlag zurückgewonnen werden.

Der Anteil an zugesetztem, wirksamen Thrombin hängt von verschiedenen Faktoren ab. Hinsichtlich der Verfahrensbedingungen beschleunigt eine hohe Thrombinkonzentration die Fibrinbildung, so daß bei hohen Thrombinkonzentrationen das Reaktionsgemisch nach relativ kurzer Zeitspanne tiefgefroren werden muß, um noch einen ausreichenden Fibrinogengehalt zu gewährleisten. Hinsichtlich des Endproduktes und dessen verschiedenen Anwendungen wird für ein Wundversorgungsmaterial ein relativ hoher Thrombingehalt gewünscht, um gemeinsam mit dem zusätzlichen Fibrinogenangebot die Haemostase zu beschleunigen. Zur Herstellung eines Wundversorgungsmaterials, das auch ohne nachträgliche Zugabe von gerinnungsaktiven Enzymen und Faktoren eine befriedigende haemostatische Wirkung zeigt, kann die Fibrinogen-Lösung mit relativ hohen Thrombinmengen , beispielsweise mit 20 bis 30 und mehr Einheiten Thrombin pro 1 ml Lösung versetzt werden. Für andere Anwendungsfälle, etwa als Trägermaterial für Wirkstoffe wie Antibiotika, ist der Thrombingehalt des Endproduktes von geringerer Bedeutung; in diesem Fall genügt ein geringer Thrombinzusatz, der ausreicht, das vorgelegte Fibrinogen innerhalb angemessener Zeitspannen zu dem angestrebten hohen Anteil von mehr als 50 % zu Fibrin umzusetzen. In diesem Falle reichen katalytisch wirksame Mengen Thrombin von wenigstens 0,1 Einheiten, vorzugsweise von etwa 5 bis 10 Einheiten pro 1 ml Fibrinogen-Lösung aus.

Sofern un-physiologisch hohe Salz- und/oder Stabilisierungsmittel-Konzentrationen nicht vorliegen, erzeugt Thrombin aus Fibrinogen in wässrigem Medium polymerisierbare Fibrinmonomere. Die Natur des aus diesen Monomeren gebildeten Polymerisates hängt von verschiedenen Faktoren ab. Würde man den Fibronogen- und Thrombin-haltigen Ansatz sich selbst überlassen, so wäre nach etwa 4 bis 6 Stunden das gesamte Fibrinogen umgesetzt, und man erhält ein steifes Gel, das beim Schütteln zu Fibrinfäden zusammenfällt. In diesem Fall ist die Polymerisation über die $\alpha$- und $\gamma$-Ketten der Fibrinmonomere erfolgt. Würde man den erhaltenen Niederschlag lyophilisieren, so würde man ein hartes, brüchiges Produkt erhalten, das wegen seiner geringen mechanischen Festigkeit und Biegsamkeit, sowie wegen seiner verminderten Löslichkeit und verzögerten Abbaubarkeit als Wundversorgungsmaterial weniger geeignet ist.

Die ausschließliche Polymerisation der Fibrinmonomere ergibt ein lösliches, wenig stabiles Polymerisat, das überwiegend durch Wasserstoffbrücken-Bindungen, elektrostatische Wechselwirkungen, Hydratation und dgl. stabilisiert ist. Eine höhere Stabilität resultiert aus der Bildung kovalenter Bindungen zwischen benachbarten Fibrinmolekülen. Hierzu ist bekanntlich die Anwesenheit von Faktor XIII erforderlich, der seinerseits in Anwesenheit von $CaCl_2$ durch Thrombin aktiviert wird. In Abhängigkeit von den Herstellungsbedingungen enthält festes oder gelöstes Fibrinogen praktisch immer einen kleineren oder größeren Anteil an Faktor XIII. Häufig wird die Abscheidung des Rohfibrinogens aus dem Plasma unter solchen Bedingungen durchgeführt, daß zusammen mit dem Rohfibrinogen auch Faktor XIII abgeschieden wird. Die Erzeugung von kovalenten Bedingungen kann in den verschiedenen Seitenketten benachbarter Fibrinmoleküle stattfinden, welche in unterschiedlichem Ausmaß zur Längs- und Quervernetzung des Fibrinpolymerisates beitragen.

Im Rahmen der Erfindung ist erkannt worden, daß ein überwiegend längs vernetztes, lösliches Fibrinpolymerisat erhalten werden kann, wenn die Erzeugung kovalenter Bindungen rechtzeitig abgebrochen wird, da die zur Quervernetzung erforderlichen kovalenten Bindungen langsamer gebildet werden, als die zur Längsvernetzung erforderlichen kovalenten Bindungen. Das In-Gang-Kommen der Quervernetzung macht sich durch einen deutlichen Viskositätsanstieg des Ansatzes bemerkbar. Zur Kontrolle der abgeschlossenen $\gamma$-Kettendimerisierung kann die Viskosität der Fibrinogen/Fibrinmonomeren-Lösung überwacht werden. Bricht man die Reaktion nicht rechtzeitig ab, so kann unter der Wirkung der $\alpha$-Kettenpolymerisierung die Viskosität den 10-fachen Wert der Viskosität der Ausgangslösung erreichen. Zur Erzeugung eines erfindungsgemäßen Trockenpräparates ist eine Fibrinogen/Fibrinmonomeren-Lösung dann gut geeignet, wenn ihre Viskosität den doppelten Wert der Ausgangsviskosität erreicht hat, weil dann die $\gamma$-Kettendimerisierung weitgehend abgeschlossen ist, und die $\alpha$-Kettenpolymerisierung noch nicht nennenswert eingesetzt hat. Abhängig von der Thrombin- und der Faktor XIII-Konzentration ist die Verdoppelung der Ausgangsviskosität in der Regel 30 bis 40 min nach der Thrombinzugabe erreicht.

Für viele Anwendungsfälle, insbesondere als Wundversorgungsmaterial, ist ein überwiegend längs vernetztes Fibrinpolymerisat erwünscht, da dieses leichter löslich ist, eine größere physiologische Aktivität aufweist und große Mengen Thrombin gebunden enthält und deshalb für eine schnelle Blutgerinnung angestrebt wird. Weiterhin kann gerade solches überwiegend längs vernetztes Fibrin, nämlich überwiegend nur $\gamma$-kettendimerisiertes Fibrin in der Lösung verschäumt oder durch Zusatz von Glutaraldehyd nach Bedarf vernetzt werden.

Als Anhaltspunkt für das Ausmaß der Quer- und Längsvernetzung des Fibrinpolymerisates kann die $\alpha$-Polymeren- und $\gamma$-Dimeren-Bildung gemessen werden. Hierzu wird Fibringemisch in Natrium-Laurylsulfat-haltigem Puffer mit Zusätzen an Mercaptoethanol gelöst und in Polyacrylamidgelen elektrophoretisch getrennt.

Sofern ein überwiegend längs vernetztes Fibrinpolymerisat angestrebt wird, soll die Reaktionsdauer des Ansatzes nach der Thrombinzugabe auf maximal 40 min beschränkt werden. Vorzugsweise ist nach der Thrombinzugabe eine Reaktionsdauer von etwa 10 bis 30 min vorgesehen. Unter diesen Bedingungen wird ein Fibrinpolymerisat erhalten, das vorwiegend $\gamma$-Ketten-Dimer enthält und im wesentlichen frei von $\alpha$-Kettenpolymeren ist. Ergänzend kann die Fibrinbildung in Gegenwart von SH-Gruppenblockierenden Substanzen wie beispielsweise Jodazetat durchgeführt werden. Hierdurch wird der Anteil an Fibrin, das in Form von Fibrinmonomeren vorliegt, gesteigert.

Unabhängig davon kann für manche Anwendungsfälle auch ein höheres Maß an Quervernetzung erwünscht sein, insbesondere wenn das Trockenpräparat einen besonders hohen Fibringehalt von mehr als 80 Gew.-% aufweisen soll. Ein hohes Maß an Quervernetzung ergibt ferner eine dichtere Struktur und erhöht die Verweildauer des Trockenpräparates im Gewebe. Sofern ein höheres Maß an Quervernetzung angestrebt wird, kann die Reaktionsdauer nach der Thrombinzugabe auch mehr als 40 min betragen und auf mehrere Std. ausgedehnt werden. Auch die nach der Gefriertrocknung der dabei entstehenden Gele erhaltenen Trocken-Präparate sollen von der vorliegenden Erfindung umfaßt sein. In diesem Falle ist es wünschenswert, eine Zerstörung der Gelstruktur zu vermeiden, weshalb der Ansatz unmittelbar nach der Thrombinzugabe und kurzzeitigem Umrühren in die Gefriertrocknungsform gegeben wird, für die vorgesehene Reaktionsdauer unbewegt bei Raumtemperatur gehalten wird und darauf schnell abgekühlt wird.

Darüberhinaus kann die Vernetzung des Fibrinpolymerisates auch durch die Zugabe von Vernetzungsmitteln gesteuert werden. Zu geeigneten Vernetzungsmitteln gehören beispielsweise zweiwertige, brücken-bildende Stoffe wie etwa SPDP, nämlich N-Succin-imidyl-3-(2-pyridyl-dithio)-propionate, oder zweiwertige, mit den vorhandenen Aminogruppen Peptidbindungen erzeugende organische Substanzen wie etwa Formaldehyd, Glutaraldehyd, Malonyldialdehyd, Adipinsäure und/oder deren Derivate. Im Hinblick auf ihre hohe Wirksamkeit kann die zugesetzte Menge an Vernetzungsmittel recht gering gehalten werden und beispielsweise nur 0,1 bis 5 % des gesamten Ansatzes betragen. Gut bewährt hat sich beispielsweise ein Zusatz von 3 ml Glutaraldehyd zu einem 100 ml Ansatz. Eine solche Vernetzung erhöht auch die Zug- und Reißfestigkeit des gebildeten Trockenpräparates.

Erfindungsgemäß wird das in dem Trockenpräparat enthaltene Fibrin somit in situ in einem wässrigen, Fibrinogen und Thrombin enthaltendem Medium erzeugt. Das auf diese Weise gebildete Fibrin erweist sich als wesentlich nativer, löslicher und reiner als beispielsweise das durch CaCl$_2$-Fällung aus Humanplasma gefällte und abgetrennte Fibrin gemäß US-PS 3 523 807. Das letztere Material ist praktisch unlöslich, enthält Einschlüsse von anderen Plasmaproteinen und ist deshalb für die Anwendung als Wundversorgungsmaterial weniger geeignet.

Vorzugsweise wird die in situ - Bildung von Fibrin unter solchen Bedingungen durchgeführt, daß das erhaltene Fibrinpolymerisat kovalente Bindungen zwischen benachbarten Fibrinmolekülen aufweist. Hierzu soll das Reaktionsgemisch wenigstens katalytisch wirksame Mengen an Faktor XIII enthalten; vorzugsweise soll 1 ml Reaktionsgemisch wenigstens 0,5 bis 1 Einheiten Faktor XIII enthalten. Besonders bevorzugt ist ein Fibrinpolymerisat dieser Art, dessen kovalente Bindungen überwiegend zu einer Längsvernetzung geführt haben, d.h., der Anteil der eine Quervernetzung verursachenden kovalenten Bindung an der Gesamtzahl aller kovalenten Vernetzungen soll weniger als 2 % betragen. Als Anhaltspunkt für das Ausmaß der Quervernetzung kann die Gelelektrophorese dienen. Das besonders bevorzugte Fibrinpolymerisat ist wegen seiner γ-Kettendimerisierung überwiegend längsvernetztes Fibrin. Größere Komplexe können durch nachträgliche Polymerisierung und/oder Vernetzung erzeugt werden.

Nachdem die Fibrinbildung, -polymerisation und -vernetzung ausreichend weit fortgeschritten ist, wird der Ansatz tiefgefroren. Hierzu wird rasch, beispielsweise innerhalb von 5 min auf eine Temperatur unterhalb -20$^o$C, vorzugsweise auf -40$^o$C oder noch tiefere Temperaturen abgekühlt. Die Wahl der Abkühlungstemperatur ist nicht von kritischer Bedeutung, da Thrombin und die anderen Gerinnungsfaktoren praktisch unterhalb 0$^o$C keine nennenswerte Aktivität aufweisen.

Das Material wird solange auf der Abkühlungstemperatur gehalten, bis der gesamte Ansatz durch und durch zu einem massiven Eiskörper gefroren ist. Je nach Größe des Ansatzes hat sich hierzu eine Verweildauer von etwa 20 bis 30 min bei - 40°C in der Gefrierform gut bewährt.

Anschließend wird die Trocknung unter bekannten Bedingungen durchgeführt. Hierzu bringt man den oder die Eiskörper in eine Vakuumkammer und erwärmt so langsam, daß die verdunstende Flüssigkeit stets abgeführt und an einer Kühlfalle niedergeschlagen werden kann, so daß sich das Tiefkühlprodukt niemals verflüssigt. Sofern man bei einem Unterdruck von weniger als 1 bis 60 Pa arbeitet, ist der Trocknungsvorgang in etwa 3 bis 8 Stunden beendet.

Das erhaltene Produkt ist eine lockere Masse mit schaum-, filz- oder vliesartiger Struktur. Bei der Prüfung unter dem Mikroskop erkennt man bei 200-facher Vergrößerung ein ineinander verwobenes Netzwerk aus feinen Fasern. Das erhaltene Vlies hat das Volumen des ursprünglichen Flüssigkeitsansatzes, und weist innerhalb des gesamten Vliesvolumens eine homogene, einheitliche Zusammensetzung auf. Zumeist liegt das spez. Gewicht der Vliesstruktur zwischen etwa 0,1 und 0,5 g/cm$^3$. Sofern ein noch lockereres und leichteres Material angestrebt wird, kann der flüssige Ansatz vor dem Einfüllen in die Gefriertrocknungsform verschäumt werden. Hierzu wird ein inertes Treibmittel wie etwa Stickstoff oder Kohlendioxid in die Flüssigkeit eingeblasen. Durch Zugabe oberflächenaktiver Mittel kann eine bestimmte Porengröße des gebildeten Schaumes eingestellt werden.

Die äußeren Abmessungen des nach der Gefriertrocknung erhaltenen Vlieses sind an den jeweiligen Anwendungszweck angepaßt. Für die Anwendung als Wundversorgungsmaterial eignet sich gut ein etwa 6 bis 20 mm starkes Vlies mit einer Länge von etwa 3 bis 12 cm und einer Breite von 1 bis 12 cm. Soll das Trockenpräparat vorzugsweise als Wirkstoffdepot dienen und in das Gewebe implantiert oder in Knochenhöhlen eingesetzt werden, so haben sich stückige Formen bewährt, beispielsweise Kugeln mit einem Durchmesser von etwa 1 bis 3 cm Würfel, mit einer Kantenlänge von etwa 1 bis 3 cm oder Scheiben, Zäpfchen und dgl. mit ähnlichen Abmessungen. Zur Erzielung dieser äußeren Abmessungen wird der flüssige Ansatz zweckmäßigerweise in entsprechend geformte Gefriertrocknungsformen eingefüllt.

Nach Abschluß des Trocknungsverfahrens wird das Trockenpräparat vorsichtig an einem Rand vom Formboden gelöst und abgezogen. Das erhaltene Vlies wird kurzzeitig auf einer Al-Folie abgelegt, soweit erforderlich, auf die gewünschten Maße zurechtgeschnitten, und daraufhin in eine eingesenkte Plastikform eingelegt und die letztere mit einer Al-Folie verschlossen. Daraufhin kann die Verpackung samt Inhalt sterilisiert werden, beispielsweise mit Röntgenstrahlung (Dosis: 3 min lang 3000 rad). Das feuchtigkeitsdicht und steril verpackte Produkt ist bei Raumtemperatur praktisch unbegrenzt lagerfähig, ohne nennenswert an Aktivität zu verlieren.

Wie bereits ausgeführt, bestehen für das erfindungsgemäße Trockenpräparat verschiedene Anwendungsmöglichkeiten, und die Zusammensetzung des Trockenpräparates kann gezielt an den jeweiligen Anwendungszweck angepaßt werden.

Nach der allgemeinsten Ausführungsform der Erfindung soll das Vlies neben Thrombin in zumindest katalytisch wirksamen Mengen im wesentlichen aus etwa 10 bis 95 Gew.-% Fibrin und etwa 5 bis 90 Gew.-% Fibrinogen bestehen. Unter "zumindest katalytisch wirksamen Mengen" wird ein Thrombingehalt von etwa 0,1 bis 10 Einheiten, vorzugsweise von 3 bis 8 Einheiten pro 1 cm$^3$ Vliesmaterial verstanden. Bei diesen "Einheiten" handelt es sich um die in der Fachwelt üblichen "NIH-Einheiten" (gemäß dem Standard des National Institut of Health der U.S.A.). Bei einem Fibringehalt von weniger als 10 Gew.-% überwiegt die Fibrinogen-Natur des Trockenpräparates, so daß ein brüchiges Material mit unzureichender mechanischer Festigkeit vorliegt. Der Fibringehalt soll daher wenigstens 10 Gew.-%, vorzugsweise wenigstens 30 Gew.-% des Trockenpräparates ausmachen. Fibringehalte von mehr als 95 Gew.-% erfordern Reaktionsbedingungen, die ein festes, physiologisch wenig aktives Material liefern, das vom Organismus nur schwer abgebaut werden kann. Der Fibringehalt soll deshalb nicht mehr als 95 Gew.-%, vorzugsweise nicht mehr als 70 Gew.-% des Vliesgewichtes ausmachen. Ein Trockenpräparat mit einem Fibringehalt von etwa 20 bis 30 Gew.-% und mit einem Fibrinogengehalt von etwa 80 bis 70 Gew.-% liefert nach dem Anfeuchten mit Körperflüssigkeit ein dem natürlichen Wundverschlußmaterial besonders ähnliches Präparat und wird daher besonders bevorzugt.

Sofern das erfindungsgemäße Trockenpräparat vor allem als blutstillendes und wundheilendes Wundversorgungsmaterial eingesetzt werden soll, soll das Vlies überwiegend aus Fibrinogen bestehen. In diesem Falle hat sich ein Fibringehalt von etwa 10 bis 40 Gew.-% und ein Fibrinogengehalt von etwa 60 bis 90 Gew.-% gut bewährt. Das Wundversorgungsmaterial soll beim Befeuchten mit dem Wundsekret die Flüssigkeit aufnehmen, sich anlösen und eine hoch viskose, klebrige Paste bilden, die an der Wundfläche haftet, dem Ausströmdruck des Blutes standhält und die Gerinnungsenzyme des kontaktierenden Blutes aktiviert. Zu dieser Aktivierung werden mit dem Trockenpräparat vorzugsweise zusätzlich gerinnungsfördernde Substanzen, gefäßaktive Stoffe, Gerinnungsfaktoren und dgl. angeboten. Diese Komponenten können bereits in die zur Erzeugung des Trockenpräparates dienende Lösung eingebracht und gemeinsam mit dieser tiefgefroren und lyophilisiert werden. Vorteilhaft an dieser Alternative ist, daß die Komponenten in höchst feiner Verteilung innerhalb des Trockenpräparates vorliegen, was deren Wirksamkeit noch weiter steigert. Alternativ können diese Komponenten in Form einer pulverförmigen Wirkstoffkombination nachträglich in das im wesentlichen nur aus Thrombin, Fibrin und Fibrinogen bestehende Trockenpräparat eingearbeitet werden. Dies erlaubt die Anwesenheit von Wirkstoffen, deren Aktivität durch die Gefriertrocknung beeinträchtigt wird, und/oder außergewöhnlich hohe Thrombinkonzentrationen, welche die sonstige Verfahrensführung wegen der beschleunigten Fibrinbildung einschränken würden.

Ein geeignetes pulverförmiges biochemisches Substrat zu akzelerierten Haemostase und optimierten biochemischen Regelung des Wundverschlusses, das pulverförmig auf dem vorgebildeten, erfindungsgemäßen Trockenpräparat aufgebracht werden kann, ist im einzelnen in der europäischen Patentanmeldung Nr. 8 111 0615.2 vom 18. Dezember 1981 beschrieben. Mit der Bezugnahme auf diese europäische Patentanmeldung soll deren Inhalt, soweit erforderlich, auch zum Bestandteil der vorliegenden Unterlagen gemacht werden. Dieses pulverförmige, biochemische Substrat ist im Hinblick auf eine optimierte Aktivierung des exogenen und/oder endogenen Gerinnungssystems sowie unter Berücksichtigung einer Vielzahl physiologischer und pathologischer Faktoren konfektioniert. Dieses Substrat enthält u.a. Fibrinogen, Thrombin, Komponenten des Prothrombinkomplexes, Proteaseinhibitoren. Je nach Applikationszweck können zusätzlich Blutplättchen-Extrakte, Antibiotika und dgl. in zweckmässigen Mischungsverhältnissen beigefügt werden. Eine bevorzugte Ausführungsform dieses Plasmaderivates besteht im wesentlichen aus 80 bis 94 Gew.-% Fibrinogen, 1 bis 10 Gew.-% Thrombin und/oder Prothrombin und 0,01 bis 3 Gew.-% Fibrinolyse-Inhibitor, enthält weniger als 0,4 Gew.-% kälteunlösliches Globulin und kann darüber hinaus zusätzlich Phospholipide, Prostaglandine, Trockenhalte- und Stabilisierungsmittel, Antibiotika und/oder Blutgerinnungsfaktoren, alle in fester, pulverförmiger Form, enthalten. Diese hochwirksame, pulverförmige Wirkstoffkombination kann in Anteilen von etwa 0,1 bis 5 Gew.-Teile auf 100 Gew.-Teile Trockenpräparat aufgebracht werden. Vorzugsweise werden diese geringen Mengen mittels eines sterilen Gasstrahls auf die Oberfläche des Trockenpräparates aufgeblasen und haften dort in ausreichender Menge. Das danach erhaltene Fibrin/Fibrinogen-Vlies kann direkt zur Wundbehandlung verwendet werden oder in einen Schnellverband (Heftpflaster) integriert werden. Auf diese Weise wird ein Wundversorgungsmaterial erhalten, das an der Oberfläche eines ausschließlich biologischen Trägermaterials eine

hochwirksame Wirkstoffkombination zur akzelerierten Haemostase und optimierten biochemischen Regelung des Wundverschlusses aufweist.

Ferner kann das erfindungsgemäße Trockenpräparat als Trägermaterial bzw. als Wirkstoffdepot für einen oder mehrere, irgendeinen Heilungsprozeß fördernde(n) Wirkstoffe(n) verwendet werden. Für diesen Anwendungszweck soll das Trockenpräparat überwiegend aus Fibrin bestehen. Ein hoher Fibringehalt gewährleistet eine hohe mechanische Festigkeit des Vlieses, eine ausreichene Verweildauer im Gewebe und die gewünschte Wirkstoff-Freisetzung über einen längeren Zeitraum hinweg. Zusätzlich kann die Anwesenheit eines die zeitliche und mengenmäßige Wirkstoff-Freisetzung steuernden Bestandteils wie etwa Collagen, vorgesehen werden. Weiterhin kann die Wirkstoff-Freisetzung durch die Art und das Ausmaß der Fibrinvernetzung beeinflußt werden. Für diesen Anwendungszweck besteht das Trägermaterial vorzugsweise aus etwa 65 bis 95 Gew.-% Fibrin und etwa 5 bis 35 Gew.-% Fibrinogen und enthält zusätzlich wenigstens einen, den Heilungsprozeß fördernden Wirkstoff. Besonders gute Ergebnisse wurden mit einem solchen Trägermaterial aus etwa 70 bis 85 Gew.-% Fibrin und etwa 15 bis 30 Gew.-% Fibrinogen erzielt. Den oder die Wirksotf(e) kann man bereits in die zur Trockenpräparat-Herstellung verwendete Lösung einarbeiten und gemeinsam mit dieser lyophilisieren oder nachträglich in das lyophilisierte Trockenpräparat einarbeiten.

Ein spezielles Anwendungsgebiet eines solchen Wirkstoffhaltigen Fibrin/Fibrinogen-Vlieses ist die Behandlung von Knochenmark-Entzündungen, weil das Trägermaterial voll resorbierbar ist und deshalb eine Nachoperation zur Entfernung des Depotmaterials nicht länger erforderlich ist. In diesem Fall ist das Trockenpräparat zweckmäßigerweise in Form von Tabletten, Kugeln, oder sonstiger geeigneter Form konfektioniert und enthält neben Antibiotika Beimischungen von Calcium, Protease-Inhibitoren und Faktor XIII in beliebiger Kombination oder zweckmäßigen Mischungsverhältnissen, um die Resorptionszeit und proteolytische Abbaubarkeit zu determinieren. Ferner kann ein Zusatz von Wachstumsfaktoren, wie z.B. Hormone und Plättchenextrakte in therapeutisch erwünschter Menge und Verteilung vorgesehen werden.

In Verbindung mit Knochenfrakturen kann es zweckmäßig sein, mit dem Wirkstoff-haltigen Trockenpräparat gezielt auch die Knochenneubildung fördernde Zusätze anzubieten. Hierzu gehören etwa natürliches, feinteiliges Knochenmaterial, wie etwa denaturiertes Knochenmehl oder lyophilisierte und pulverisierte Knochenpartikel oder synthetischer, knochenbildender Ersatzstoff, wie etwa anorganische Salze des Kaliums, Magnesiums und Calciums, insbesondere Calciumphosphat, und hier besonders bevorzugt Tricalciumphosphat. Ferner können die Kallusbildung anregende Wirkstoffe wie etwa Thrombocyten-Wachstumsfaktoren oder Hormone vorgesehen werden. Für diesen speziellen Anwendungszweck besteht das Trockenpräparat vor allem aus etwa 30 bis 45 Gew.-% Fibrin, aus etwa 4 bis 15 Gew.-% Fibrinogen, Rest natürliches feinteiliges Knochenmaterial und/oder synthetischer, knochenbildender Ersatzstoff sowie wenigstens ein antibakterieller Wirkstoff. Bei Bedarf kann dieses Trockenpräparat zusammen mit sterilisierten, lyophilisierten, homologen Knochenstücken und/oder mit autologer Spongiosa in die jeweilige Knochenhöhle eingebracht werden. Dank seiner voluminösen und biegsamen Struktur lassen sich auf diese Weise mit dem erfindungsgemäßen Trockenpräparat Knochenhöhlen beliebiger Abmessungen gut ausfüllen und abdichten. Das sich neu bildende Knochengewebe kann dann mit der allmählichen Resorption des Fibrinnetzwerkes in dieses einsprossen, wobei die gesteuerte Wirkstoff-Freisetzung über einen langen Zeitraum hinweg ein antiseptisches Milieu gewährleistet.

Mit der vorliegenden Erfindung wird somit ein Trägersystem aus einem Fibrin-Gemisch-Schaum zur Aufnahme biochemischer Stoffe und Substrate zur akzelerierten Haemostase und optimierten biochemischen Regelung des Wundverschlusses bereitgestellt. Als Grundsubstanz des Trägersystems dient ein Schaum, bestehend aus einem Gemisch von Fibrin und anderen Substanzen. Je nach den jeweils gewünschten Applikationszwecken wird dem Fibrin-Gemisch-Schaum einzeln oder in beliebiger Kombination und zweckmäßigen Mischungsverhältnissen Fibrin, Thrombin, Prothrombin, Blutplättchenextrakte, Protease-Inhibitoren etc. zugesetzt. Durch die Beimischung von Proteinen wie etwa Albumin, Lipoprotein, Fibrin, Fibronektin und Globulin kann die gewünschte Flexibilität und Stabilität des Schaumes eingestellt werden. Als Beimischung zu dem Fibrin-Gemisch-Schaum können - je nach Applikationszweck - einzeln, in beliebiger Kombination oder in zweckmäßigen Mischungsverhältnissen, insbesondere Fibrin, Thrombin, Prothrombin, Blutplättchenextrakt, Protease-Inhibitoren, Antibiotika etc. zugesetzt werden. Ferner kann als Beimischung zu dem Fibrin-Gemisch-Schaum ein Substrat oder Substrat-Komplex zur akzelerierten Haemostase und optimierten chemischen Regelung des Wundverschlusses zugesetzt werden.

Die nachstehenden Beispiele dienen zur weiteren Erläuterung der Erfindung, ohne diese einzuschränken.

Beispiel 1:

5 g Fibrinogen (mikrokristallines, durch Alkohol/Glycin-Fällung aus Humanplasma erhaltenes Präparat wie oben angegeben) werden in 100 ml 0,9 %-iger NaCl-Lösung gelöst, so daß eine Proteinendkonzentration von 50 mg/ml resultiert. Der Fibrinogen-Lösung werden unter Rühren 300 Einheiten Thrombin zugesetzt ("Topostasin" der Firma Hoffmann LaRoche AG, Grenzach-Wyhlen). Es wird noch kurzzeitig gerührt und daraufhin der gesamte Ansatz in eine Lyophilisierungsform gegossen und für weitere 20 min bei Raumtemperatur gehalten. Daraufhin wird tiefgefroren, nämlich der gesamte Ansatz einschl. der Form innerhalb von 10 min auf etwa -40°C abgekühlt. Der gebildete Eisblock wird daraufhin lyophilisiert, wozu unter Vakuum die Dampfphase kontinuierlich abgepumpt und ausgefroren wird. Man erhält ein Gemisch aus Fibrinogen und löslichem Fibrin in Form eines lockeren, zusammenhängenden Proteinvlieses, das eine gute mechanische Festigkeit und Reißfestigkeit aufweist. Neben katalytisch wirksamen Mengen Thrombin besteht dieses Trockenpräparat aus etwa 20 Gew.-% Fibrin und 80 Gew.-% Fibrinogen. An diesem Trockenpräparat wurden die nachstehenden Untersuchungen durchgeführt:

Bestimmung des Fibringehaltes:

1,0 g des Trockenpräparates werden mit 10 ml 0,9 % NaCl-Lösung aufgenommen, gerührt und von unlöslichen Anteilen getrennt. Der Überstand wird in der Gelelektrophorese unter Zusatz von Natrium-Laurylsulfat (ohne Mercaptoethanol) in Fibrinmonomere und Fibrinogen getrennt. Die Anteile werden nach Anfärben mit Coomassie-Brilliant-Blue photometrisch vermessen und bestimmt. Es resultiert ein Fibringehalt von etwa 20 % und ein Fibrinogengehalt von etwa 80 %.

Bestimmung der Fibrinogenaktivität:

1 ml der nach vorstehendem beschriebenen Verfahren erhaltenen Fibrinogen-Lösung wird mit 3 Einheiten Thrombin versetzt, das sich bildende Fibrin um einen Stab gedreht, und die verbleibende Lösung abgetrennt. Im Photometer lassen sich die Extinktionen vor und nach der Fibrinabtrennung bestimmen. Aus dem ermittelten Wert wurde der Anteil an gerinnbarem Fibrinogen zu mehr als 85 % des vorhandenen Fibrinogens errechnet.

Bestimmung der Thrombinaktivität:

1 g des Trockenpräparates werden in 10 ml 0,9 %iger NaCl-Lösung aufgenommen und bei 37°C inkubiert. Die Zeit bis zum Eintritt der Gerinnung wird gemessen. Eine Vergleichslösung mit reinem Fibrinogen und steigenden Zusätzen an Thrombin dient als Bezugsgröße zur Bestimmung der Thrombinaktivität im Lyphilisat. Im vorliegenden Fall konnte etwa 35 bis 45 Einheiten Thrombin nachgewiesen werden.

Zur Anwendung als Wundversorgungsmaterial wird das Fibrinogen/Fibrinvlies auf Abmessungen von 8 x 50 x 100 mm zurechtgeschnitten und sterilisiert. Bei Bedarf wird das Produkt auf die Wundfläche aufgelegt. Durch das Aufsaugen des Wundsekrets wird die Gerinnung des austretenden Blutes in Gang gesetzt.

Beispiel 2:

9 g Fibrinogen werden in 100 ml dest. Wasser aufgenommen und mit 300 Einheiten Thrombin versetzt. Nach dem Eingießen der Lösung in die Lyophilisierungsform wird der Fortgang der Reaktion beobachtet und bei einem merklichen Viskositätsanstieg nach ungefähr 20 min das Material sofort tiefgefroren. Durch den höheren Thrombinzusatz wird der Anteil an löslichem Fibrin auf 40 bis 50 % gesteigert.

Beispiel 3:

9 g Fibrinogen werden mit 100 ml 0,1 %-iger Collagenlösung aufgenommen und mit 1 Million Einheiten Gentamycin versetzt. Zusätzlich enthält der Ansatz noch 100 000 Einheiten Trasylol. Das so vorbereitete Gemisch wird unter Rühren mit 3000 Einheiten Thrombin versetzt und in eine Lyophilisierungsform gegossen. Nach einer Reaktionszeit von 6 Std. wird der Inhalt der Form tiefgefroren und lyophilisiert.

Beispiel 4:

5 g Fibrinogen werden in 100 ml einer 1 %-igen Albuminlösung aufgelöst und mit 3 Millionen Einheiten Gentamycin, 500 000 Einheiten Trasylol und 3000 Einheiten Thrombin versetzt. Nach Ausgießen in die Lyophilisierungsform werden 20 min später 2 ml Glutaraldehyd als Vernetzungshilfe zugegeben und der Ansatz lyophilisiert.

Beispiel 5:

9 g Fibrinogen werden in 100 ml einer 5 %-igen Albuminlösung aufgenommen. Dem Ansatz wird ein Anteil von 5 Millionen Einheiten Baycillin und 1 Million Einheiten Aprotinin zugesetzt. Nach Zugabe von 3000 Einheiten Thrombin wird das Material in eine inerte Form gegossen und nach dem Gelieren tiefgefroren. Der Anteil an Fibrin kann dadurch auf 85 % angehoben werden. Das lyophilisierte Material gibt ein Proteinvlies mit kompakter Struktur, das in Würfel geschnitten gut zur Versorgung infizierter Knochenmarkshöhlen eingesetzt werden kann.

Beispiel 6:

5 g Fibrinogen werden in 100 ml, 0,9 %-iger NaCl-Lösung, die 0,025 M $CaCl_2$ enthält, gelöst. Dem Ansatz werden 1,0 g Albumin und 500 Einheiten Faktor XIII zugesetzt. Als Antibiotikum soll das Material 1 Million Einheiten Baycillin enthalten, die der Lösung als Pulver beigegeben werden. 100 000 Einheiten Aprotinin dienen zur Verhinderung einer vorzeitigen Lyse des Wundversorgungsmaterials im Wundgebiet. Der Inhibitor wird ebenfalls als Pulver der Lösung zugesetzt. Durch die Zugabe von 30 000 Einheiten Thrombin läßt man die Bildung des Fibrins in Gang kommen, dabei befindet sich der Ansatz bereits in der Trockenform. Nach einer Reaktionszeit von 1 h wird das Material gefriergetrocknet. Der Zusatz von Faktor XIII erhöht den Vernetzungsgrad des Fibrins und fördert die Stabilität des Vlieses.

Beispiel 7:

Zu 100 ml Plasma werden 5 g Fibrinogen gegeben und unter Rühren gelöst. Nach Zusatz von 300 Einheiten Thrombin wird die nach 20 Minuten gelierende Masse in eine Gefriertrocknungsform gegeben, tiefgefroren und anschließend lyophilisiert.

Beispiel 8:

5 g Fibrinogen werden in 100 ml 0,9 %-iger NaCl-Lösung gelöst und mit 3000 Einheiten Thrombin versetzt. Nach 12 Stunden wird das Fibrin durch Rühren aus der Gelform in die Faserform überführt. Das fasrige Material wird in 5 %-iger Albuminlösung suspendiert und mechanisch zerkleinert. Nach Zusatz von 2 ml Glutaraldehyd wird der Ansatz tiefgefroren und anschließend lyophilisiert.

Patentansprüche:

1. Fibrinogen-haltiges Trockenpräparat mit einer durch Gefriertrocknung erzielten Schaum- bzw. Vliesstruktur, insbesondere als Wundversorgungsmaterial, Füllmaterial für Knochenhohlräume und/oder Trägermaterial für weitere Wirkstoffe, dadurch gekennzeichnet, daß das Vlies neben Thrombin in zumindest katalytisch wirksamen Mengen im wesentlichen aus etwa 10 bis 95 Gew.-% Fibrin und etwa 5 bis 90 Gew.-% Fibrinogen besteht.

2. Trockenpräparat nach Anspruch 1, dadurch gekennzeichnet, daß 1 cm$^3$ Vliesmaterial etwa 0,1 bis 10 Einheiten (NIH-Einheiten) Thrombin enthält.

3. Trockenpräparat nach Anspruch 1, dadurch gekennzeichnet, daß das vor allem als blutstillendes und wundheilendes Wundversorgungsmaterial vorgesehene Vlies einen Thrombingehalt von wenigstens 1 Einheit Thrombin pro 1 cm$^3$ Vliesmaterial aufweist und

etwa 10 bis 40 Gew.-% Fibrin,

etwa 60 bis 90 Gew.-% Fibrinogen, und

0 bis 1,2 Gew.-% gerinnungsfördernde Substanzen, gefäßaktive Stoffe und Gerinnungsfaktoren enthält.

0068149

4. Trockenpräparat nach Anspruch 1 oder 2,
   dadurch gekennzeichnet, daß
   das vor allem als blutstillendes und wundheilendes
   Wundversorgungsmaterial vorgesehene Vlies im wesentlichen aus 10 bis 40 Gew.-% Fibrin und 60 bis 90 Gew.-%
   Fibrinogen besteht, und in dieses Vlies nachträglich
   ein pulverförmiges angereichertes Plasmaderivat, nämlich eine Wirkstoffkombination zur akzelerierten
   Haemostase und optimierten biochemischen Regelung des
   Wundverschlusses eingearbeitet worden ist.

5. Trockenpräparat nach Anspruch 1 oder 2,
   dadurch gekennzeichnet, daß
   das vor allem als Trägermaterial vorgesehene Vlies aus
   etwa 65 bis 95 Gew.-% Fibrin sowie
   etwa 5 bis 35 Gew.-% Fibrinogen besteht und zusätzlich
   wenigstens einen, den Heilungsprozeß fördernden Wirkstoff wie etwa ein Antibiotikum oder dgl. enthält.

6. Trockenpräparat nach Anspruch 1 oder 2,
   dadurch gekennzeichnet, daß
   das vor allem als Füllmaterial für pathologische
   Knochenhohlräume vorgesehene Vlies besteht aus
   etwa 30 bis 45 Gew.-% Fibrin,
   etwa 4 bis 15 Gew.-% Fibrinogen,
   Rest natürliches feinteiliges Knochenmaterial und/oder
   synthetischer, knochenbildender Ersatzstoff sowie
   wenigstens ein antibakterieller Wirkstoff.

7. Trockenpräparat nach Anspruch 5 oder 6,
   dadurch gekennzeichnet, daß
   das Vlies zusätzlich einen, die zeitliche und mengenmäßige Wirkstoff-Freisetzung steuernden Bestandteil
   enthält

8. Trockenpräparat nach einem der Ansprüche 1 bis 7,
   <u>dadurch gekennzeichnet, daß</u>
   das Fibrin im wesentlichen nur über die $\gamma$-Ketten der
   Fibrinmonomere verknüpft ist.

9. Trockenpräparate nach einemm der Ansprüche 1 bis 8,
   <u>dadurch gekennzeichnet, daß</u>
   das Vlies zusätzlich ein oder mehrere Glykoproteine,
   nämlich Albumin, Lipoprotein, Fibronektin oder Globulin
   enthält.

10. Trockenpräparat nach einem der Ansprüche 1 bis 9,
    <u>dadurch gekennzeichnet, daß</u>
    das Vlies zusätzlich einen oder mehrere Fibrinolsyse-
    Inhibitor(en) enthält.

11. Verfahren zur Herstellung eines Trockenpräparates nach
    einem der Ansprüche 1 bis 10,
    <u>dadurch gekennzeichnet, daß</u>
    in einer Fibrinogen und Thrombin enthaltenden wässrigen
    Lösung in situ Fibrin erzeugt wird, und
    das entstehende Reaktionsgemisch tiefgefroren und
    llyophilisiert wird.

12. Verfahren nach Anspruch 11
    <u>dadurch gekennzeichnet, daß man</u>
    a) Wasser, Humanserum oder eine wässrige Salzlösung mit
       Fibrinogen versetzt;
    b) der Fibrinogen-haltigen Lösung gegebenenfalls einen
       oder mehrere Wirkstoffe, Zusätze und dergleichen
       hinzufügt;
    c) die erhaltene Lösung mit Thrombin oder einer Thrombin-
       bildenden Vorstufe versetzt;

0068149

d) das Gemisch unter üblichen Bedingungen reagieren läßt, bis sich ein Teil, jedoch nicht die Gesamtmenge Fibrinogen zu Fibrinmonomeren umgesetzt hat;

e) nach ausreichender Umsetzung tieffriert; und

f) schließlich lyophilisiert.

13. Verfahren nach Anspruch 11 oder 12,
dadurch gekennzeichnet, daß
man der Ausgangslösung pro 1 ml Lösung 10 bis 90 mg Fibrinogen und 0,1 bis 10 Einheiten Thrombin zusetzt nach der Thrombinzugabe bei Raumtemperatur 10 bis 40 min lang rührt;
das Reaktionsgemisch auf eine Temperatur unterhalb -20o C abkühlt; und
das gebildete Eis lyophilisiert, ohne daß sich erneut eine flüssige Phase bildet.

14. Verfahren nach einem der Ansprüche 11 bis 13,
dadurch gekennzeichnet, daß
die Fibrinbildung in Gegenwart von SH-Gruppen blockierenden Substanzen durchgeführt und/oder das Fibringemisch durch Zusatz von Vernetzungsmittel vernetzt wird.

15. Verfahren nach einem der Ansprüche 11 bis 14,
dadurch gekennzeichnet, daß
man das aus der Gefrierform entnommene Trockenpräparat zurechtschneidet, feuchtigkeitsdicht verpackt, und die Verpackung samt Inhalt sterilisiert.

16. Verwendung des Trockenpräparates nach einem der Ansprüche 1 bis 10,
zur akzelerierten Haemostase und optimierten biochemischen Regelung des Wundverschlusses, wozu man auf dem Fibrin/Fibrinogen-Vlies eine pulverförmige Wirkstoffkombination, enthaltend Fibrinogen, Thrombin,

0068149

Komponenten des Prothrombinkomplexes, Proteaseinhibitoren, Blutplättchenextrakt und/oder Antibiotika aufträgt.

17. Verwendung des Trockenpräparates nach einem der Ansprüche 1 bis 10,
zur Behandlung von Knochenmarksentzündungen,
wozu man das Fibrin/Fibrinogen-Vlies mit den zur
Therapie erforderlichen Wirkstoffen wie etwa Antibiotika und dgl. versieht und in den pathologischen
Knochenhohlraum einbringt.

18. Verwendung nach Anspruch 17,
wobei das Vlies teilchenförmig ausgebildet ist, etwa
in der Gestalt von Kugeln oder Tabletten.